Europäisches Patentamt

European Patent Office

Office européen des brevets

(19)

(11) Publication number: **0 248 936**
**A1**

## (12) EUROPEAN PATENT APPLICATION

(21) Application number: **86108111.5**

(22) Date of filing: **13.06.86**

(51) Int. Cl.4: **A61K 7/30 , C11D 3/00**

(43) Date of publication of application:
**16.12.87 Bulletin 87/51**

(84) Designated Contracting States:
**AT BE CH DE FR GB IT LI LU NL SE**

(71) Applicant: **Richardson GmbH**
**H.-S.-Richardson-Strasse Postfach 1661**
**D-6080 Gross-Gerau(DE)**

(72) Inventor: **Agar, T.H. Dr.**
**2, Bellingham Close**
**Heatherside Camberley Surrey(GB)**
Inventor: **Talbot, J.M.**
**12a, Avenue Road**
**Staines Middlesex(GB)**
Inventor: **Muench, W.**
**64, Pierson Road**
**Windsor Berkshire(GB)**
Inventor: **Rennie, Paul**
**21, Bramble Way**
**Ripley Surrey(GB)**

(74) Representative: **Goddar, Heinz J., Dr. et al**
**FORRESTER & BOEHMERT**
**Widenmayerstrasse 4/I**
**D-8000 München 22(DE)**

(54) Cleaning tablet for dentures and method for producing thereof.

(57) The invention relates to a cleaning tablet for automatic cleaning of dentures in aqueous solution, subdivided in two layers with different compositions, that shows a change of a pH from acidic to alkaline during the dissolving process of the tablet in water, as well as a method for producing such a cleaning tablet.

EP 0 248 936 A1

## Cleaning tablet for dentures and method for producing thereof

The invention relates to a cleaning tablet for automatic cleaning of dentures in aqueous solution, subdivided in two layers with different compositions, having a content of sodium hydrogen carbonate as the carbon dioxide producing agent, sodium polyphosphate as binder, further organic binders and acid carriers, potassium monopersulphate as oxidizing agent; surfactants in the form of alkyl or alkyl aryl sulphonates, hardened triglycerides and silicon dioxide as tabletting aids, polyethylene glycol with a molecular weight of approximately 20,000 as granulation medium, fatty acid esters of sugar as foam supporters and colouring and flavouring agents, as well as a method for producing thereof.

The DE-Y-23 57 720 discloses a cleaning tablet that is subdivided in two layers with different compositions. These two layers are dissolving in water with very different rates, so that there is a two-phase cleansing of the denture. This solution shows a pH during the whole process of dissolving and cleansing of between 6.5 bis 7.0. Although this known cleaning tablet already shows very good cleansing properties, there are still some problems especially with respect to the germ killing ability.

Thus, the problem of the invention is to provide a cleaning tablet for dentures that shows superior cleansing properties and superior germ killing ability than the known tablet.

According to the invention this problem is solved by a cleaning tablet of the above mentioned kind which is characterized by a change of the pH from acidic to alkaline during the dissolving process of the tablet in water.

The invention further relates to an improved method for producing a cleaning tablet according to the invention that is characterized in that sodium bicarbonate, sodium perborate monohydrate, tartaric acid, sodium tripolyphosphate, sulphamic acid, polyethylene glycol (20 M) and ethylene diamine tetraacetate are granulated; that the indicated quantities of such granulates and of the other ingredients are tumble-mixed to form one mixture for the first layer and one mixture for the second layer; that the mixture for the second layer is compressed at a low pressure and that then the mixture for the first layer is compressed to the second layer at a high pressure to form a two-phase tablet.

Preferred embodiments of the cleaning tablet and the method of producing thereof are subject of the respective subclaims.

The cleaning tablet according to the invention shows a characteristic pH swing from acidic to alkaline and a greater generation of oxygen than the commercial two-phase tablet.

The destaining, plaque removal and antimicrobial activity of the cleaning tablet according to the invention has been compared with the commercial formulation in a series of tests. The improved tablet was at least twice as effective at stain removal than commercial tablets. Both tablets removed 43 % of artificial plaque by dry weight. In suspension tests, those tablets rapidly killed Staphylococcus aureus, Streptococcus mutans and Pseudomonas aeruginosa. The improved tablet was considerably more active against Escherichia coli and Candida albicans, which is of particular importance, since Candida albicans is the only resident oral microorganism likely to be pathogenic to denture wearers. Against in vitro mixed plaques, the improved tablet was more active against Escherichia coli, Staphylococcus aureus and particularly Streptococcus mutans. Overall, the cleaning tablet according to the invention represents significant improvement over the existing formula.

Moreover the composition and the method of producing according the invention leads to a tablet that is much more consistent with respect to manufacture at high speed and high pressure and to chemical composition.

Typically, a cleaning tablet according to the invention has the following composition in its first layer:

25 - 35 % by weight sodium bicarbonate,
20 - 30 % by weight tartaric acid,
10 - 20 % by weight potassium monopersulphate,
10 - 13 % by weight sulphamic acid,
3 - 15 % by weight disodium pyrophosphate,
3 - 5 % by weight sodium carbonate,
2 -4 % by weight polyethylene glycol (20 M)
0 - 3 % by weight sodium sulphate,
1 - 2 % by weight flavour blend,
1 -2 % by weight silicon dioxide,
0.3 - 0.8 % by weight sodium dodecyl benzene sulphonate
and the following composition in its second layer:
25 - 45 % by weight sodium perborate monohydrate,

25 - 35 % by weight potassium monopersulphate,

10 - 25 % by weight sodium bicarbonate,

7 - 18 % by weight sodium tripolyphosphate,

2 - 4 % by weight ethylene diamine tetraacetate,

2 - 4 % by weight sodium carbonate,

2 - 3 % by weight polyethylene glycol (20 M),

1 - 3 % by weight silicon dioxide,

1 - 2 % by weight flavour blend,

1 - 2 % by weight fatty acid esters of sugar,

0.3 - 0.8 % by weight hardened triglycerides,

0.3 - 0.5 % by weight sodium dodecyl benzene sulphonate,

0.2 - 0.4 % by weight succinate detergents,

0.05 - 0.15 % by weight colouring agents.

Among the above mentioned ingredients present in both layers of the tablet according to the invention, sodium bicarbonate serves as gaseous carbon dioxide supply, while potassium monopersulphate is an oxidizing agent supplying gaseous oxygen to the solution.

The organic acids, i. e. tartaric acid and sulphamic acid in the first layer have an antimicrobial effect, detaching scale from dentures, and are providing the acidity in the first phase of cleansing.

The content of ethylene diamine tetraacetate is softening and demineralizing. Disodium pyrophosphate in the first layer and sodium tripolyphosphate in the second layer are detergent builders, attacking the denture plaque by their surface activity. Sodium dodecyl benzene sulphonate and the succinate detergents, both being effective surfactants, are improving the cleansing activity. The fatty acid esters of sugar are supporting the action of the surfactants.

Sodium perborate monohydrate is another oxidizing agent providing at the same time for the alkaline conditions in the second phase of the cleansing process.

Sodium carbonate is a stability aid, silicon dioxide as well as the hardened triglycerides are tabletting aids and sodium sulphate acts as a filler.

The preferred flavour blend consists of 20 - 25 % of a commercial peppermint oil carried on maltodextrin powder and the colouring agents in the second layer are preferably blue dyes.

The combination of ingredients causing the above mentioned pH swing according to the present invention is providing for an enhanced activity of each of the two layers compared to the two-phase tablet known from DE-Y-23 57 720.

A typical compressed tablet common in the pharmaceutical industry would carry active substances within a compression base. Such bases are those that are known to compress well into hard, non friable tablets. Thus the technology of compression can be developed for the base, then active substances added. This cannot be in a tablet according to the invention that is expected to dissolve rapidly, generate and sustain a high rich foam, direct a pH swing, evolve carbon dioxide then oxygen, cleanse, wet and sanitise/deodorise.

Therefore many ingredients must fulfill at least two functions. As an example, the compounds chosen to produce gaseous oxygen must be suitable to compression without brittleness, must evolve oxygen at an optimal delivery rate over a selected period and leave alkaline rendering residues to the solution. Another example is the use of silicon dioxide: firstly it acts as a glidant-lubricant such that powder flows via feed-tubes and hoppers to the tablet presses; secondly, within the tablet itself the dry, amorphous silicon dioxide acts to dry out sticky detergents which would otherwise cause sticking of tablets to hot compression punch metal faces. The content of the silicon dioxide must be chosen optimal, any increase causing brittle, chipping tablets and unacceptable cloudiness of the solution.

Producing cleaning tablets according to the invention is quite complex for the following reasons:

1. As mentioned above, the formulation does not permit the inclusion of pharmaceutical tabletting compression aids. Thus the tablet is formed by the skilled techno logy of compressing detergents, oxygen generators and phosphate builders.

2. Forming a solid, permanent union between the two layers is difficult to accomplish since the second, "blue" layer must be hard enough to survive as a pre-tablet yet soft enough to allow key-binding of the following first, "white" layer. Therefore finely balanced formulations and respective pressure are necessary.

3. Produced tablets must be sufficiently hard to survive harsh packaging process yet sufficiently porous to rapidly dissolve in water.

Further features and advantages of the invention can be gathered form the following description of a performance example.

First of all, sodium bicarbonate, sodium perborate monohydrate, tartaric acid, sodium tripolyphosphate, sulphamic acid, polyethylene glycol (20 M) and ethylene diamine tetraacetate are separately granulated by fluidising in a hot air bed at 60 - 65 °C for 30 minutes. Such granulates are then tumble mixed with the other ingredients to produce a "first layer" mixture and a "second layer" mixture, wherein the "first layer" mixture has the following composition:

|  |  | % by weight |
|---|---|---|
| sodium bicarbonate | | 30.00 |
| tartaric acid | | 23.00 |
| potassium monopersulphate | | 16.00 |
| sulphamic acid | | 11.00 |
| disodium pyrophosphate | | 8.20 |
| sodium carbonate | | 3.90 |
| polyethylene glycol (20 M) | | 2.60 |
| sodium sulphate | | 2.00 |
| peppermint powder | | 1.50 |
| silicon dioxide | | 1.30 |
| sodium dodecyl benzene sulphonate | | 0.50 |

and the "second layer" mixture has the following composition:

|  | % by weight |
|---|---|
| sodium perborate monohydrate | 30.00 |
| potassium monopersulphate | 28.00 |
| sodium bicarbonate | 13.34 |
| sodium tripolyphosphate | 10.00 |
| sodium bicarbonate/colour | 4.00 |
| Trilon B | 3.00 |
| sodium carbonate | 3.00 |
| polyethylene glycol (20 M) | 2.50 |
| silicon dioxide | 2.00 |
| peppermint powder | 1.50 |
| Wasag ester 7 | 0.70 |
| Wasag ester 15 | 0.70 |
| hardened triglycerides | 0.50 |
| sodium dodecyl benzene sulphonate | 0.40 |
| succinate detergent | 0.30 |
| Blue Lake No. 1 | 0.06 |

4

A tablet is produced by compressing in a HORN rotary tabletting press of 39 stations. Compressing is in two stages: Initially the "second layer", blue mixture is compressed to very low pressure (10 kN per tablet by way of tamping. The "first layer", white mixture is then instilled and pressed to 70 kN per tablet. In this way a tablet of 4 grammes is produced being 2.7 grammes blue and 1.3 grammes white. Outputs are in the order of 45,000 tablets per hour.

The features disclosed in the foregoing description and in the following claims may, both separately and in any combination thereof, be material for realising the invention in diverse forms thereof.

## Claims

1. A cleaning tablet for automatic cleaning of dentures in aqueous solution, subdivided in two layers with different compositions, having a content of sodium hydrogen carbonate as the carbon dioxide producing agent, sodium polyphosphate as lime binder, further organic lime binders and acid carriers, potassium monopersulphate as oxidizing agent, surfactants in the form of alkyl or alkyl aryl sulphonates, hardened triglycerides and silicon dioxide as tabletting aids, polyethylene glycol with a molecular weight of approximately 20,000 as granulating medium, fatty acid esters of sugar as foam supporters and colouring and flavouring agents characterized by a change of the pH from acidic to alkaline during the dissolving process of the tablet in water.

2. A cleaning tablet according to claim 1, characterized by an additional content of sodium perborate monohydrate and the use of tartaric acid and sulphamic acid as organic acid carriers.

3. A cleaning tablet according to claim 2, characterized by the following composition of the first layer:
25 - 35 % by weight sodium bicarbonate,
20 - 30 % by weight tartaric acid,
10 - 20 % by weight potassium monopersulphate,
10 - 13 % by weight sulphamic acid,
3 - 15 % by weight disodium pyrophosphate,
3 - 5 % by weight sodium carbonate,
2 - 4 % by weight polyethylene glycol (20 M)
0 - 3 % by weight sodium sulphate,
1 - 2 % by weight flavour blend,
1 - 2 % by weight silicon dioxide,
0.3 - 0.8 % by weight sodium dodecyl benzene sulphonate
and the following composition in its second layer:
25 - 45 % by weight sodium perborate monohydrate,
25 - 35 % by weight potassium monopersulphate,
10 - 25 % by weight sodium bicarbonate,
7 - 18 % by weight sodium tripolyphosphate,
2 - 4 % by weight ethylene diamine tetraacetate,
2 - 4 % by weight sodium carbonate,
2 - 3 % by weight polyethylene glycol (20 M),
1 - 3 % by weight silicon dioxide,
1 - 2 % by weight flavour blend,
1 - 2 % by weight fatty acid esters of sugar,
0.3 - 0.8 % by weight hardened triglycerides,
0.3 - 0.5 % by weight sodium dodecyl benzene sulphonate,
0.2 - 0.4 % by weight succinate detergents,
0.05 - 0.15 % by weight colouring agents.

4. A method for producing a cleaning tablet according to one of the preceding claims, characterized in that sodium bicarbonate, sodium perborate monohydrate, tartaric acid, sodium tripolyphosphate, sulphamic acid, polyethylene glycol (20 M) and ethylene diamine tetraacetate are granulated; that the indicated quantities of such granulates and of the other ingredients are tumble-mixed to form one mixture for the first layer and one mixture for the second layer: that the mixture for the second layer is compressed at a low pressure and that then the mixture for the first layer is compressed to the second layer at high pressure to form a two-phase tablet.

5. A method according to claim 4, characterized in that the granulation is done by fluidising the substances in a hot air bed at 60 - 65 °C.

6. A method according to claim 4 or 5, characterized in that the low pressure is applied at approximately 10 kN/tablet and the high pressure at approximately 70 kN/tablet.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 028 005 (G. GERGELY) <br> * Whole document * | 1-6 | A 61 K 7/30 <br> C 11 D 3/00 |
| Y,D | DE-A-2 357 720 (KUKIROL) <br> * Whole document * | 1-6 | |
| Y | EP-A-0 151 203 (RICHARDSON GmbH) <br> * Whole document * | 1-6 | |
| A | DE-A-2 527 534 (JOHNSON & JOHNSON) <br> * Claims 1-9 * | 1-4 | |
| A | DE-B-2 647 364 (KIKIDENT) <br> * Whole document * | 1-4 | **TECHNICAL FIELDS SEARCHED (Int. Cl.4)** |
| A | FR-A-2 290 187 (RECKITT & COLMAN) <br> * Claims 1-8 * | 1-4 | A 61 K <br> C 11 D |

---

The present search report has been drawn up for all claims

| Place of search | Date of completion of the search | Examiner |
|---|---|---|
| THE HAGUE | 29-01-1987 | FISCHER J.P. |